# FASCICULE DE BREVET EUROPEEN

(11) **EP 3 484 614 B1**
(45) Date de publication et mention de la délivrance du brevet: **18.08.2021**
(21) Numéro de dépôt: 17735606.0
(22) Date de dépôt: 11.07.2017
(51) Int. Cl.: B01J 20/291, C07C 61/29, C08L 93/00

(54) **ORGANOGELS PERMETTANT DE REDUIRE ET CONTROLER L'EVAPORATION DE LIQUIDES ORGANIQUES VOLATILS**
ORGANOGELE ZUR ERMÖGLICHUNG DER REDUZIERUNG UND STEUERUNG DER VERDAMPFUNG VON FLÜCHTIGEN ORGANISCHEN FLÜSSIGKEITEN
ORGANOGELS ALLOWING REDUCTION AND CONTROL OF THE EVAPORATION OF VOLATILE ORGANIC LIQUIDS

(30) Priorité: 12.07.2016 FR 1656701
(43) Date de publication de la demande: 22.05.2019
(73) Titulaire: Centre National de la Recherche Scientifique (CNRS), 75016 Paris (FR); Université Paul Sabatier Toulouse III, 31400 Toulouse (FR)
(72) Inventeur: TER-HALLE, Alexandra, 31450 Baziege (FR); PEREZ, Emile, 31770 Colomiers (FR); GIRAUD, Isabelle, 31000 Toulouse (FR); FRANCESCHI, Sophie, 31320 Pechbusque (FR)
(74) Mandataire: Regimbeau
(86) Numéro de dépôt international: PCT/EP2017/067446
(87) Numéro de publication internationale: WO 2018/011226

(56) Documents cités:
- CN-A- 102 212 172
- FR-A1- 2 988 619
- Mehmet Z. Haznedaroglu ET AL: "Preparation and Evaluation of a Novel Organogel Formulation of Salvia tomentosa Mill. Essential Oil", Latin American Journal of Pharmacy, 1 juin 2013 (2013-06-01), pages 845-851, XP055354729, Extrait de l'Internet: URL:https://www.researchgate.net/profile/A ysu_Yurdasiper/publication/259450859_Prepa ration_and_Evaluation_of_a_Novel_Organogel _Formulation_of_Salvia_tomentosa_Mill_Esse ntial_Oil/links/57063e4208aea3d28020ce4e/P reparation-and-Evaluation-of-a-Novel-Organ ogel-Formulation-of-Salvia-tomentosa-Mill- Essential- [extrait le 2017-03-14]

## Description

La présente invention concerne des compositions chimiques sous la forme d'organogel comprenant un liquide organique, comprenant ou consistant en un liquide organique volatil, et au moins un acide résinique, leur procédé de préparation, et leurs utilisations afin de réduire et contrôler l'évaporation du liquide organique volatil.

De manière générale, les organogels sont des matériaux semi-solides consistant en un liquide organique ou une huile, immobilisés par un réseau tridimensionnel résultant de l'auto-assemblage d'un organogélifiant polymèrique ou de faible masse moléculaire. L'organogélifiant de faible masse moléculaire correspond à une petite molécule organique, ou un mélange de petites molécules organiques, capable de gélifier à de faibles proportions une large gamme de liquides organiques. Par faible masse moléculaire, on entend une masse moléculaire inférieure à 1000 g/mol, et plus particulièrement inférieure à 500 g/mol.

Le terme « organogel » signifie un gel dans lequel le liquide (diluant) est un support organique ou un solvant organique (par opposition à l'eau).

Le terme "organogel thermoréversible" est synonyme d' "organogel physique" et désigne un organogel dont la structure réseau est due à des interactions de faible énergie et thermiquement instables telle que la liaison hydrogène (par opposition à des liens solides, thermiquement stables tels que des liaisons covalentes) et peut, par conséquent, être transformé par chauffage en un liquide à écoulement libre. Lors du refroidissement en dessous d'une température caractéristique (Tgel), la reformation des liaisons faibles permet le rétablissement de la structure de gel solide.

Au sens de la présente invention, le terme organogélifiant désigne une molécule organique capable de gélifier, à de faibles proportions, une large gamme de liquides organiques (Cf: Terech P. & Al Low molecular mass gelators of organic liquids and the properties of their gels Chemical Reviews 1997, 97, (8), p.3133-3159 ou Abdallah D.J. & Al Organogels and low molecular mass organic gelators Advanced materials 2000,12, (7), p.1237 -1243 ou Terech P. « Low molecular weight organogelators in Specialist Surfactants I.D. Robb (Ed), Blackie Academic and Professional, Glasgow. 1996, p.208-268). Des oragnogels visant à capturer des huiles essentielles volatiles sont divulgués dans la demande de brevet Française FR2988619 ainsi que dans un article par Haznedaroglu et al. dans "Latin American Journal of Pharmacy" (2013), pages 845-851.

On entend par liquide organique, un liquide hydrophobe dont les molécules ou les parties de molécules présentent une certaine répulsion vis-à-vis des molécules d'eau. Un liquide organique peut être volatil ou non.

Le liquide organique peut être un solvant organique au sens chimique du terme. Le terme « solvant organique » se réfère aux solvants qui sont des composés organiques qui contiennent des atomes de carbone. Les solvants permettent de dissoudre les réactifs et d'amener les réactifs en contact. Ils ne réagissent pas chimiquement avec le composé dissous. Les solvants peuvent aussi être utilisés pour extraire les composés solubles d'un mélange.

Dans la suite de la présente description on utilisera le terme liquide organique qui pourra englober la notion fonctionnelle de solvant organique.

Par volatilité on entend la capacité d'une substance, généralement liquide, à température ambiante (de l'ordre de 20°C) et à pression atmosphérique (environ 760 mm Hg) à se vaporiser. La volatilité peut être définie comme le rapport de la fraction molaire de la phase gazeuse sur la fraction molaire de la phase liquide dans une situation d'équilibre, pour un liquide donné.

Les gels, largement utilisés en cosmétique et dans l'industrie alimentaire ou pétrolière, sont généralement des solutions diluées de polymères, de protéines, de substances inorganiques comme de la silice ou les argiles dans l'eau ou des liquides organiques. Les gélifiants sont nécessaires à la formation et au maintien de ces gels.

Récemment, un intérêt croissant s'est développé pour les organogélifiants de faible masse moléculaire. Cette situation est motivée non seulement par les nombreuses applications potentielles des organogels obtenus à partir de ces gélifiants particuliers, mais également par le fait que ces systèmes présentent d'intéressantes propriétés d'auto assemblage. Dans ces organogels, tout comme pour les autres systèmes gélifiés, l'agent gélifiant forme un réseau continu tridimensionnel qui immobilise le liquide et l'empêche de couler. Contrairement à leurs analogues polymériques ou inorganiques, l'architecture du réseau résulte de l'auto-assemblage des organogélifiants de faible masse moléculaire par des forces non covalentes, telles que des liaisons hydrogènes, des forces de Van Der Walls ou encore des interactions donneur-accepteur. Généralement le réseau tridimensionnel est constitué d'un assemblage de molécules d'organogélifiant en fibrilles ou en cristallites interconnectés.

Dans les domaines cosmétique, pharmaceutique ou alimentaire les organogels présentent l'avantage de pouvoir contenir des ingrédients ou principes actifs pour faciliter l'introduction, et la stabilité desdits ingrédients ou principes actifs dans des compositions.

Plus particulièrement dans le domaine cosmétique, plusieurs solutions ont été envisagées afin d'introduire, de protéger, voire même de vectoriser certains ingrédients lorsque leur stabilité pouvait être remise en cause par le milieu de formulation, le procédé de fabrication, ou lorsqu'il est préférable que le principe actif ne soit libéré qu'à un endroit donné. La pénétration des principes actifs à travers les différentes couches de la peau peut également être améliorée.

Dans le cas particulier de gélification de liquides organiques volatils, tels que par exemple des huiles essentielles ou des hydrocarbures, il a été observé que la gélification seule ne permet pas toujours de réduire ou de contrôler leur volatilisation ce qui fait qu'une composition de type organogel d'un liquide organique volatil peut parfois perdre en quelques heures la quasi-totalité dudit liquide organique par volatilisation.

De nombreuses industries mettent en œuvre des liquides organiques volatils, que ce soit dans le domaine de la cosmétique, de la parfumerie, de l'industrie phytosanitaire ou pharmaceutique et aussi bien évidemment dans l'industrie des hydrocarbures par exemple.

Les huiles essentielles (H. E.) sont obtenues par diverses méthodes d'extraction d'une matière végétale botaniquement définie. Les huiles essentielles sont des mélanges complexes comprenant les composés odoriférants et volatils d'une plante.

En particulier, de par leurs propriétés antibactériennes, les huiles essentielles trouvent de nombreuses applications en médecine humaine ou vétérinaire, dans le domaine phytosanitaire pour la lutte contre les stress biotiques des cultures par exemple, dans la cosmétique, la parfumerie ou encore l'agroalimentaire. Cependant de par leur volatilité très marquée et difficilement contrôlable, leur utilisation à grande échelle est limitée. Pour certains domaines d'application cette forte volatilité des huiles essentielles pose problème. Elles se volatilisent rapidement et ont donc une activité réduite dans le temps.

Dans le domaine des arômes et parfums, le contrôle de la volatilité des mélanges parfumants, désodorisants et odoriférants, en général constitués de liquides organiques volatils, peut se faire pas inclusion ou encapsulation dans une matrice neutre permettant un relargage ralenti. Une telle encapsulation est cependant complexe technologiquement, souvent trop séquestrante et onéreuse. Dans le domaine phytosanitaire du contrôle biologique, l'utilisation de phéromones d'insectes ou d'huiles essentielles se développe et constitue une alternative écologique à la lutte contre les insectes ravageurs. Cependant la formulation de ces composés généralement sous la forme de liquides organiques lipophiles et fortement volatils est difficile car il convient de bien contrôler et maîtriser leur diffusion afin d'obtenir l'effet recherché.

Des solutions visant à limiter la volatilité de liquides organiques, tels qu'en particulier les huiles essentielles par exemple, existent et consistent en l'encapsulation de celles-ci dans une matrice polymérique de type protéique ou polysaccharidique. De telles solutions sont cependant très complexes, onéreuses à mettre en œuvre, et surtout ne permettent pas de contrôler et moduler à façon la volatilisation.

C'est ainsi qu'il apparaît un besoin de limiter et contrôler la volatilité des liquides organiques, par exemple les huiles essentielles, afin d'éviter leur évaporation trop rapide ou non souhaitée ou encore afin de pouvoir moduler à dessein cette volatilité et leur évaporation selon les applications envisagées.

La Demanderesse a mis en évidence qu'il était possible de limiter et contrôler la volatilité et l'évaporation des liquides ou solvants organiques via leur gélification et leur mélange avec des résines terpéniques, en particulier des composés de type acides résiniques ou acides terpéniques.

Les inventeurs ont en effet mis en évidence que l'ajout d'une faible proportion en masse de résines naturelles à un solvant ou liquide organique volatil gélifié permet de réduire son évaporation à température ambiante.

Il a aussi été démontré qu'il y avait de plus un effet de synergie avec la formation d'un organogel. C'est-à-dire qu'un liquide organique gélifié par un organogélifiant combiné à l'ajout de résine réduit très fortement la volatilité du liquide organique. Cette technologie permet de réduire mais surtout de contrôler la volatilisation d'un liquide organique, telle qu'une huile essentielle par exemple, à une température donnée en modulant les proportions gélifiant/composés résiniques.

Lorsque les liquides organiques sont gélifiés sous la forme d'une composition de type organogel, la présence de composés de type résines terpéniques, en particulier des composés de type acides résiniques ou acides terpéniques - outre la facilité de manipulation conférée par la solidification desdits liquides organiques - permet de limiter et contrôler la volatilité de ces liquides organiques.

C'est ainsi un premier objet de la présente invention que de fournir une composition sous la forme d'un organogel d'un liquide organique comprenant au moins un acide résinique. Cette composition n'est pas une composition adhésive comme par exemple divulguée dans la demande de brevet Chinoise CN102212172.

L'acide résinique peut être sous la forme acide ou sous la forme estérifiée.

Dans un deuxième mode de réalisation l'organogel est composé d'un liquide organique, comprenant ou consistant en un liquide organique volatil, et d'un organogélifiant.

Selon un troisième mode de réalisation le liquide organique, comprenant ou consistant en un liquide organique volatil, est choisi dans le groupe constitué par les huiles essentielles, les solvants organiques, les phéromones à chaînes grasses, les hydrocarbures et leurs mélanges.

De manière préférentielle, le liquide organique est un liquide organique volatil et est choisi dans le groupe constitué par les huiles essentielles, les liquides solvants organiques volatils, les phéromones à chaînes grasses, les hydrocarbures et leurs mélanges.

Selon un quatrième mode de réalisation le liquide organique est un liquide organique volatil et est choisi dans le groupe constitué par l'huile essentielle d'eucalyptus, l'huile essentielle de lavande, l'huile essentielle de thym, l'huile essentielle de géranium, l'huile essentielle de citronnelle, l'huile essentielle de menthe poivrée, l'huile essentielle de sauge et leurs mélanges.

Selon un cinquième mode de réalisation le liquide organique comprend un liquide organique volatil tel que défini ci-avant et un liquide organique non volatil choisi dans le groupe des huiles végétales, des huiles minérales, des huiles d'intérêt cosmétique (émollientes) et leurs mélanges.

Au sens de la présente invention, le terme « volatil » ou « volatile » concernant le liquide organique s'entend d'un liquide dont la pression de vapeur saturante à 20°C est supérieure à 0,01 mm de Hg, préférentiellement elle est comprise entre 0,01 et 20, de manière plus préférentielle entre 0,01 et 10 , plus préférentiellement entre 0,01 et 5 (en mm Hg à 20°C). 1 mm de Hg correspond à 133,32 Pa.

La notion de volatilité repose sur une définition précise qui est la pression de vapeur saturant (ou tension de vapeur). C'est la pression à laquelle la phase gazeuse d'une substance est en équilibre avec sa phase liquide ou solide, à une température donnée, dans un système fermé. La pression de vapeur saturante est liée à la tendance des molécules à passer de l'état liquide (ou solide) à l'état gazeux. A titre d'information, la pression de vapeur saturante de l'eau à 20°C est de 17.5 mm Hg ; celle du butane est de 1650 mm Hg ; celle du propanol est de 18 mm Hg.

Les huiles végétales sont de préférence choisies parmi l'huile de Tournesol, l'huile de colza, l'huile de soja et leurs mélanges.

Selon un sixième mode de réalisation de l'invention, l'organogélifiant est choisi dans le groupe constitué par les cires végétales, les cires animales, les cires de paraffine, les dérivés d'acide gras, les dérivés de sels d'acide gras et plus particulièrement l'acide 12-hydroxystéarique, utilisés seuls ou en mélange.

Ainsi la présente invention vise des compositions selon les différents modes de réalisation ici décrits à l'exclusion des compositions adhésives.

Selon un septième mode de réalisation, la quantité en poids d'organogélifiant dans l'organogel est comprise entre 1 et 30% ; plus particulièrement entre 2 et 20%, plus particulièrement encore entre 10 et 15%.La proportion massique d'organogélifiant contrôle la dureté, la tenue thermique et conjointement à l'acide résinique la volatilisation du liquide gélifié.

Dans un mode de réalisation particulier la cire végétale ou animale est choisie dans le groupe constitué par la cire de Candelilla, la cire de Carnauba, la cire de graine de tournesol, la cire d'abeille, la cire de son de riz, la cire de canne à sucre, la cire de pépins de raisin, la cire de jojoba, la cire de graine de sorgho, la cire de ouiricuri, la cire de feuilles de pois, la cire de feuilles de pommes de terre, la cire d'Esparto, la cire d'Ocotillo, la cire de Balanophora, la cire d'attier, la cire de seigle de mer, la cire du laurier-cerise, la cire de chêne, la cire de mimosa, la cire de fleur d'oranger, la cire de jasmin, la cire de rose, la cire de chanvre, la cire d'abricot, la cire de tubéreuse, la cire d'orange, la cire d'amande douce la cire d'aiguilles de pin.
La cire végétale peut être une cire issue des parties aériennes de plantes, feuilles, tiges, fleurs ou fruits.

La cire de Candelilla est particulièrement adaptée car bien connue. La cire de candelilla est obtenue à partir d'un arbuste dénommé *Euphorbia antisyphilitica* originaire du Nord Mexique. Cette cire dure naturelle est principalement utilisée par l'industrie cosmétique et pharmaceutique pour la fabrication des sticks. Elle est également utilisée en agroalimentaire. Elle est composée principalement d'hydrocarbures (environ 45%), d'acides gras (environ 20%), d'alcools gras, d'esters d'acides gras et comprend aussi des résines (environ 5 % chacun). La cire de Candelilla naturelle comprend au moins un acide résinique triterpénique ou un ester d'un tel acide résinique triterpénique. La cire de Candelilla naturelle peut comprendre un ou des acides résiniques diterpéniques.
Les résines présentent dans la cire de Candelilla comprennent des esters d'acide triterpéniques (oléanique, ursolique, oléanonique, ursonique et moronique).
Il existe sur le marché une cire de Candelilla de synthèse , ou substitut de Cire de Candelilla qui comprend une paraffine, de la cire de carnauba et des terpénoïdes. On peut en particulier citer le Candelilla Wax Substitute 6700 comprenant cire de Copernicia Cerifera, cire de Helianthus Annuus , Polyethylene, alcools C30 - C50, Cire d'acide Montanique, résine de Shorea Robusta, Hydrogenated Copolymer de Styrene/ ethyl Styrene/Indene hydrogéné.

La cire de Carnauba est aussi bien adaptée. C'est une cire végétale qui provient d'un palmier originaire du Brésil, le Copernicia Cerifera. Cette cire est très utilisée dans l'industrie, du fait de son haut point de fusion parmi les cires végétales commercialisées.
Elle possède de bonnes propriétés émulsifiantes et une bonne capacité de liaison aux huiles et augmente ainsi le point de fusion lors de la formation de gels avec l'huile. Elle est composée majoritairement (environ 80%) en esters d'acides gras, d'alcools gras (environ 15%), d'acides gras et d'hydrocarbures (environ 5%).
Une autre cire végétale convenable, dont la composition est proche de celle du Carnauba, est la cire de Tournesol qui est obtenue à partir de l'huile de tournesol par filtration à froid.

De manière particulière la cire est la cire de Candellila.

Selon l'invention l'acide résinique est choisi dans le groupe constitué par l'acide abiétique, l'acide néoabiétique, l'acide dehydroabiétique, l'acide palustrique et leurs mélanges.

Selon un mode dé réalisation divulgué, l'acide résinique est de manière plus générale choisi dans le groupe constitué par les acides diterpéniques.

Selon un mode dé réalisation de l'invention, l'acide résinique est choisi dans le groupe constitué par les acides triterpéniques, les esters d'acide triterpénique et leurs mélanges. Les esters d'acide triterpénique pourront être un ester d'acide oléanique, un ester d'acide ursolique, un ester d'acide oléanonique, un ester d'acide ursonique ou un ester d'acide moronique.

Selon un mode de réalisation divulgué, l'acide résinique est choisi dans le groupe constitué par les acides triterpéniques, les esters d'acide triterpénique, les acides diterpéniques ainsi que leurs mélanges.

Selon un autre mode de réalisation divulgué, l'acide résinique est sous la forme d'une résine végétale ou d'un mélange de résines végétales.

Les résines végétales sont des substances naturelles sécrétées par certains végétaux.

Le terme résineux est souvent utilisé pour désigner les conifères. Il existe en dehors des conifères de nombreux autres végétaux sécrétant des résines.

Les résines sont excrétées hors des cellules végétales. Elles se différencient des latex qui se maintiennent à l'intérieur des parois cellulaires.

Les conifères résineux produisent de la colophane. La résine de colophane contient des acides résiniques dont la nature et les proportions varient suivant l'espèce.

La résine de colophane est composée à 90% d'un mélange d'acides organiques de la famille des diterpènes, appelés acides résiniques.

Selon un autre mode de réalisation divulgué la résine végétale est choisie dans le groupe constitué par la résine de pin, la résine de colophane, la résine de Sondarac, la résine Dammar, la gomme mastic et leurs mélanges.

Selon un autre mode de réalisation de l'invention la quantité en poids d'acide résinique dans l'organogel est comprise entre 0.5 et 10%.

Un acide résinique est un acide terpénique non volatil présent dans les résines des plantes et plus particulièrement dans celle des pins. Ce sont des agents protecteurs et de préservation du bois qui sont produits par les cellules épithéliales parenchymateuses qui entourent les conduits de résine dans les forêts de conifères tempérées.

Chimiquement, les acides résiniques se forment par combinaison de molécules à deux ou trois carbones avec des unités isoprèniques pour former des structures mono, sesquiterpène-, diterpène voire triterpène). Les acides résiniques ont deux groupes fonctionnels, un groupe carboxyle et des doubles liaisons.

Les acides résiniques selon l'invention pourront être choisis dans le groupe consistant en les acides diterpéniques et les acides triterpéniques seuls ou en mélanges ainsi que leurs esters.

Il est notable de remarquer que lorsque l'organogélifiant est une cire végétale, en particulier des cires obtenues par extraction via immersion dans un mélange d'eau bouillante et d'acide, et ce de manière plus ou moins artisanale, il est courant qu'elle contienne traces de résines végétales naturelles contenant des acides résiniques et il convient d'en tenir compte dans la quantité d'acide résinique totale dans l'organogel.

Aussi la quantité d'acide résinique ajoutée dans l'organogel pourra être modulée en fonction de la quantité déjà présente, le cas échéant, dans l'organogélifiant, en particulier une cire lorsque celle-ci est utilisée comme organogélifiant.
Ainsi, quand un acide résinique est naturellement présent dans l'organogélifiant (par exemple pour la cire de Candelilla), aucun ajout supplémentaire d'acide résinique n'est nécessaire. On veillera donc à ne tenir compte que de la quantité d'acide résinique présente dans l'organogel formé par la gélification du liquide organique, comprenant ou consistant en un liquide organique volatil.

C'est aussi un autre objet de la présente invention que de viser l'utilisation d'une composition sous forme d'organogel selon la présente invention pour réduire la volatilité du liquide organique, comprenant ou consistant en un liquide organique volatil, contenu dans l'organogel.

Dans un mode de réalisation particulier de l'invention, l'utilisation selon la présente invention vise l'utilisation d'une composition sous forme d'organogel dans lequel le liquide organique, comprend ou consiste en, un hydrocarbure afin de limiter les risques d'inflammation et/ou d'explosion.

La présente invention vise aussi l'utilisation d'une composition selon l'invention dans laquelle le liquide organique gélifié comprend, ou consiste en, au moins une huile essentielle volatile ou un mélange d'huiles essentielles pour la diffusion contrôlée de cette huile essentielle ou de ce mélange.

C'est aussi un objet de la présente invention que de viser l'utilisation d'une composition selon l'invention dans laquelle le liquide organique, comprenant ou consistant en un liquide organique volatil, est une phéromone, en particulier une phéromone à chaîne grasse ou un mélange de phéromones, en particulier de phéromones à chaine grasse, pour la diffusion contrôlée de cette phéromone ou de ce mélange de phéromones.

La présente invention vise aussi l'utilisation d'une composition selon l'invention dans laquelle le liquide organique, comprenant ou consistant en un liquide organique volatil, comprend un parfum pour la diffusion contrôlée de ce parfum.

La présente invention vise aussi l'utilisation d'une composition selon l'invention dans laquelle le liquide organique, comprenant ou consistant en un liquide organique volatil, est choisi dans le groupe des liquides insecticides ou insectifuges, anti-fongiques, anti-viraux ou anti-bacteriens pour la diffusion contrôlée de ce liquide organique.

L'invention vise aussi une formulation insecticide ou insectifuge comprenant une composition selon l'invention, dans laquelle le liquide organique, comprenant ou consistant en un liquide organique volatil, est doté de propriétés insecticides ou insectifuges.

L'invention vise aussi une formulation antibactérienne comprenant une composition selon l'invention, dans laquelle le liquide organique, comprenant ou consistant en un liquide organique volatil, est doté de propriétés antibactériennes.

L'invention vise aussi une formulation antivirale comprenant une composition selon l'invention, dans laquelle le liquide organique, comprenant ou consistant en un liquide organique volatil est doté de propriétés antivirales.

Les compositions selon l'invention pourront présenter une consistance allant d'un liquide très visqueux à un solide compact et ce en fonction de la proportion d'organogélifiant dans l'organogel.

Dans un aspect avantageux, la proportion de l'organogélifiant est comprise entre 1 % et 30 % en poids par rapport au poids total de l'organogel.

Selon l'invention, l'organogélifiant de faible masse moléculaire correspond à une petite molécule organique capable de gélifier à de faibles proportions une large gamme de liquides organiques. Par faible masse moléculaire au sens de la présente invention, on entend une masse moléculaire inférieure à 1000 g/mol, et plus particulièrement inférieure à 500 g/mol.

Dans un aspect avantageux de l'invention, l'organogélifiant sera choisi parmi les cires végétales ainsi que leurs mélanges.

L'organogélifiant pourra être choisi dans le groupe constitué par les cires végétales, les cires animales, les cires de paraffine, les esters d'acides gras, les acides gras et leurs dérivés ou sels métalliques monovalents, divalents ou trivalents, et plus particulièrement l'acide 12-hydroxystéarique, utilisés seuls ou en mélange.

Les cires animales et végétales sont généralement des mélanges d'esters gras d'acides gras, d'alcools gras, d'acides gras et d'hydrocarbures de très longues chaines (> C30) .

Selon la présente invention, l'organogélifiant est préférentiellement choisi parmi les dérivés d'acides gras éventuellement substitués ainsi que leurs sels métalliques monovalents, ou leurs esters.

Un organogélifiant convenant particulièrement bien à la mise en œuvre de la présente invention est l'acide 12-hydroxystéarique.

Un organogélifiant convenant particulièrement bien à la mise en œuvre de la présente invention est la cire de Carnauba.

Un organogélifiant convenant particulièrement bien à la mise en œuvre de la présente invention est la cire de Candelilla.

Le processus de gélification consiste à former un gel à partir d'un liquide. Dans un cas particulier selon l'invention le liquide organique à gélifier est une huile ou un mélange d'huiles, comprenant ou consistant en une huile ou un mélange d'huiles volatiles.

L'agent de gélification appelé organogélifiant est capable de s'auto-organiser pour former des filaments ou des cristallites. Ces fibres ou ces cristallites s'enchevêtrent ou s'interconnectent pour former une organisation en trois dimensions. Ce réseau emprisonne le liquide organique et l'empêche de couler. D'un point de vue macroscopique le liquide est devenu « solide ». La gélification est un moyen réversible de créer un matériau à partir d'un liquide, mais en ne modifiant nullement sa composition chimique. L'auto-organisation de l'organogélifiant consiste en des interactions non covalentes telles que les liaisons hydrogènes, les complexes π-π ou les forces de van der Waals.

Les organogélifiants sont capables de gélifier toute sorte de liquides organiques même à des concentrations massiques relativement faibles (typiquement quelques pourcents en masse). L'acide 12-hydroxyoctadecanoïque (ou acide 12-hydroxystéarique, HSA) est un organogélifiant qui gélifie en formant un réseau 3D de fibres enchevêtrées. Les cires de carnauba (Car) et de candelilla (Can) sont aussi des organogélifiants, mais qui gélifient en formant un réseau interconnecté de cristallites.

Dans le cadre de la présente invention, le liquide organique, comprenant ou consistant en un ou plusieurs liquides organiques volatils, susceptible d'être gélifié sous forme d'organogel est liquide à température ambiante, c'est-à-dire à 25°C et sous pression atmosphérique de 1 atm.

Ils présentent un caractère lipophile. Ainsi, et de manière non exhaustive on peut citer les huiles végétales, les huiles minérales, les huiles émollientes, les huiles siliconées, les huiles fluorées, les huiles essentielles, les phéromones à chaînes grasses, les hydrocarbures, les parfums, ainsi que tout ingrédient ou principe actif volatil ou non, et liquide à température ambiante et pression atmosphérique, de même que toutes les combinaisons de ces liquides organiques.

Selon l'invention, le maintien de l'organogel est dû à la formation d'un réseau tridimensionnel constitué d'un assemblage de molécules d'organogélifiant en fibrilles qui s'auto-assemblent en fibres et faisceaux de fibres entrecroisées ou de cristallites qui immobilisent le liquide organique au sein du réseau formant ledit organogel obtenu.

La stabilisation du liquide organique gélifié est ainsi assurée.

Est divulgué également un procédé de préparation d'une composition sous la forme d'un organogel comprenant les étapes de :
(a) Préparation d'un mélange comprenant un organogélifiant, un liquide organique, comprenant ou consistant en un liquide organique volatil, et liquide à température ambiante, et au moins un acide résinique,
(b) chauffage afin de solubiliser tous les ingrédients,
(c) refroidissement à température ambiante du mélange afin de former un organogel.

L'organogel est ainsi préparé en mélangeant le gélifiant avec un liquide organique volatil, comprenant ou consistant en un liquide organique volatil, et au moins un acide résinique jusqu'à obtention d'une solution liquide ("sol").

Dans un mode de réalisation divulgué l'étape (a) de préparation du mélange est réalisée avec un organogélifiant comprenant naturellement un acide résinique (par exemple la cire de Candelilla). Dans un tel mode de réalisation, cette étape consiste ainsi à préparer un mélange comprenant un organogélifiant comprenant au moins un acide résinique, avec un liquide organique, comprenant ou consistant en un liquide organique volatil, et liquide à température ambiante. Selon un autre mode de réalisation divulgué l'étape (a) consiste à préparer un mélange comprenant un organogélifiant comprenant au moins un acide résinique, avec un liquide organique, comprenant ou consistant en un liquide organique volatil, et liquide à température ambiante, et au moins un acide résinique additionnel non naturellement présent dans l'organogélifiant.

Lorsque la solution refroidit, il se forme à partir d'une certaine température un gel qui fige le liquide organique. Cette température correspond à la température de transition sol-gel (Tgel) qui est une caractéristique physique du couple organogélifiant / liquide organique.

Dans un aspect avantageux de l'invention, l'étape a) est réalisée à une température comprise entre 30 et 50 °C. On prendra soin de contrôler cette température afin d'éviter l'évaporation du liquide organique qui peut comprendre ou consister en un liquide organique volatil et ce afin d'empêcher l'évaporation lors de la fabrication de l'organogel.

La synergie résine/gélification permet de réduire de manière contrôlée la volatilité, tout en solidifiant le liquide, ce qui permet une meilleure manipulation et un meilleur stockage. Cette forme gélifiée, ouvre de nouvelles perspectives dans de nombreux domaines (sanitaires, phytosanitaires, cosmétiques).

L'invention permet aussi le contrôle de la volatilisation d'un hydrocarbure volatil (eg. kérosène) ce qui permet de limiter les risques d'inflammation ou d'explosion durant son transport ou son stockage et ce sans affecter ses propriétés thermiques

Les compositions d'organogel selon la présente invention sont destinées à être utilisées pour la préparation de compositions dont l'utilité dépend de la nature et des propriétés du liquide organique comprenant ou consistant en un liquide organique volatil, plus particulièrement lorsque le liquide organique est un liquide organique volatil ou un mélange de liquides organiques volatils.

Il a été noté par ailleurs que la gélification d'un liquide organique comprenant ou consistant en un liquide organique volatil, par la cire de paraffine ou paraffine en tant qu'organogéliant permet de limiter la volatilité et l'évaporation dudit liquide organique volatil et ce sans ajout de résine ou d'acide résinique.

C'est ainsi un autre objet de la présente description que de fournir une composition sous la forme d'un organogel d'un liquide organique et de paraffine.

L'organogel est constitué d'un liquide organique, comprenant ou consistant en un liquide organique volatil, et d'une paraffine, ou mélange de paraffines, en tant qu'organogélifiant.

Le liquide organique, comprenant ou consistant en un liquide organique volatil, peut être choisi dans le groupe constitué par les huiles essentielles, les solvants organiques, les phéromones à chaînes grasses, les hydrocarbures, les huiles végétales et leurs mélanges.

La paraffine est un mélange d'alcanes à longues chaînes linéaires ou ramifiés. On parle ainsi plus de paraffines que de paraffine au sens individuel car plusieurs types de paraffines existent.

La paraffine utilisable dans le cadre de la présente invention, permettant de gélifier et réduire la volatilité d'un liquide organique, comprenant ou consistant en un liquide organique volatil, et ce sans apport de résines ou dérivés résiniques, est préférentiellement choisie dans le groupe constitué par les paraffines essentiellement constituées d'alcanes linéaires (n-alcanes) en C20 à C40 et présentant une température de fusion comprise entre 50 et 65°C. Il s'agit de paraffines solides à température ambiante, ie 20°C.

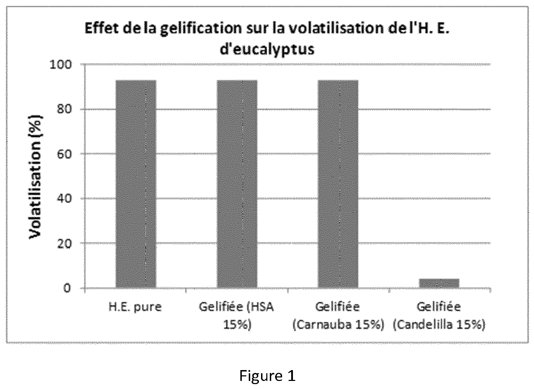

Figure 1 : Résidu à 5 heures après une expérience d'ATG à 30°C de l'effet de la gélification d'une huile essentielle d'eucalyptus.
FIGURE 2 : Effet de la gélification sur la volatilisation de l'H.E. Eucalyptus (15% massique d'organogélifiant).

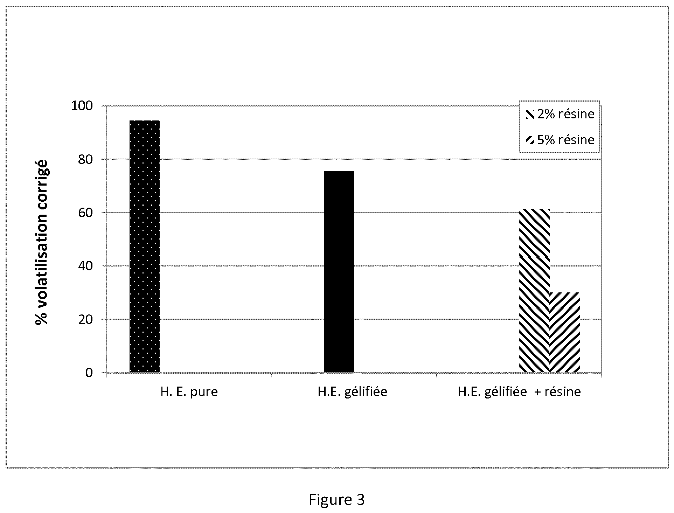

Figure 3 : Effet de l'ajout de résine de colophane (2 ou 5 %) sur H.E Eucalyptus gélifiée par C. Carnauba (15% massique)

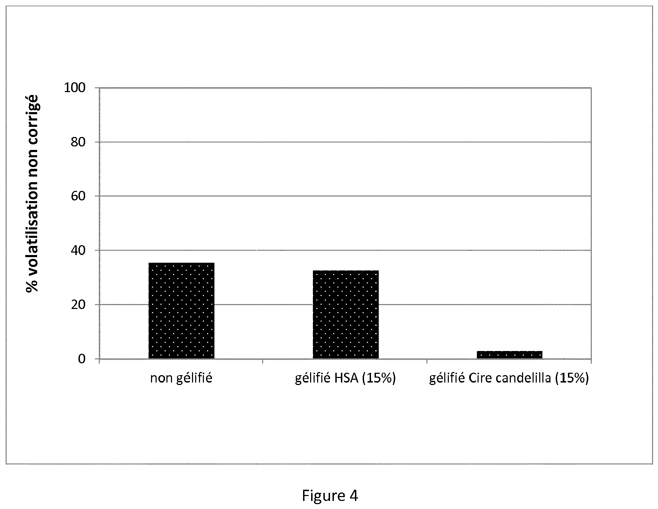

Figure 4 : Effet de la gélification (HSA 15 % ou Cire Candélilla) sur un mélange huile soja / H.E. Eucalyptus (50-50%).

### EXEMPLES

### Préparation de l'organogel.

Des concentrations fixes de gels sont obtenues en ajoutant des quantités pesées d'organogélifiant (cire végétale et/ou acide 12-hydroxystéarique) additionné ou non d'acides résiniques sous la forme de résine végétale ou d'acide résinique pur, à différentes huiles essentielles d'eucalyptus, de thym ou de lavande. Le mélange est chauffé à une température supérieure à la température de gélification généralement de l'ordre de 23 à 40°C environ. Cette température est variable selon la nature et quantité des ingrédients de l'organogel. La solution est refroidie à température ambiante et un gel est obtenu. L'état de gel est confirmé par le test du flacon retourné.

Le Tableau 1 résume les conditions de préparation de différents organogels.

**Tableau 1**

| Code | Composé volatil | organogélif iant | Tdegel (°C) | ΔHdegel J g⁻¹ | Tgel (°C) | ΔHgel J g⁻¹ |
|---|---|---|---|---|---|---|
| AH61 | H. E. Eucalyptus | HSA 7% | 23 | 1,9 | 23,8 | 1,3 |
| AH61 | H. E. Eucalyptus | HSA 15% | 37,5 | 7,7 | 41,6 | 3,6 |
| AH61 | H. E. Thym | Car 15% | Pas visible | | 35,9 | 2 |
| AH61 | H. E. Thym | HSA 15% | Plusieu rs max à 31 ; 37,5 et 52°C | | 2 max à 27, 47 | |
| AH61 | H. E. Lavande | Car 15% | Pas visible | | 2 max à 37, 51,4 | |
| AH61 | H. E. Lavande | HSA 15% | 33,6 | 9,8 | 2 max proch es 39,5 | 8,4 |
| AH67 | Eucalyptol | HSA 15% | 26,4 | 3,3 | 27,2 | 2,2 |
| AH67 | | Hent | 30,2 | 2,6 | 36,2 | 4 |
| AH67 | | Can 15% | 28 | 2,5 | 33,4 | 3,3 |
| AH67 | H.E thym | Can 15% | Peu de signal | | | |
| AH67 | H.E Lavande | Can 15% | Peu de signal | | | |
| LL06 | H.E. eucalyptus | Hent 15% | 27,4 | 3,8 | 2 max proch es 33,1 | 4,1 |
| | | Colophane 2% | | | | |
| LL06 | H.E. eucalyptus | Colophane 2% | | | | |
| LL06 | H.E. eucalyptus | | | | | |
| LL06 | H.E. eucalyptus | HSA 15% | 29,8 | 2,6 | 26,8 | 1,7 |
| | | Colophane 2% | | | | |
| LL06 | H.E. eucalyptus | Can 15% | 31 | 5 | 32,8 | 1,8 |
| LL06 | H.E. eucalyptus | Car 15% | Peu de signal | | 2 max à 34,1 26,2 | 1,8 0,7 |
| AH89 | H.E. eucalyptus | Hent 15% | 30,8 | 1,8 | Signal large 32,6 | 2 |
| | | Acide abiétique 2% | | | | |

| | | | | | | |
|---|---|---|---|---|---|---|
| Hent : Hentriacontane Car : Cire de Carnauba Can : Cire de Candelilla | | | | | | |

D'autres organogels ont été préparés de manière similaire avec des mélanges d'huile essentielle et d'huile de soja et/ou en utilisant d'autres organogélifiants tels que l'acide stéarique, le stéarate de sodium, la cire de graines de tournesol, la cire de paraffine, la cire d'aiguilles de pin ou la cire d'abeille.

L'agent de gélification appelé organogélifiant est capable de s'auto-organiser pour former des filaments ou des cristallites. Ces fibres ou ces cristallites s'enchevêtrent ou s'interconnectent pour former une organisation en trois dimensions. Ce réseau emprisonne le liquide organique et l'empêche de couler. D'un point de vue macroscopique le liquide est devenu « solide ». La gélification est un moyen réversible de créer un matériau à partir d'un liquide, mais en ne modifiant nullement sa composition chimique. L'auto-organisation de l'organogélifiant consiste en des interactions non covalentes telles que les liaisons hydrogènes ou les forces de van der Waals.

Les organogélifiants sont capables de gélifier toute sorte de liquides organiques même à des concentrations massiques relativement faibles (typiquement quelques pourcents en masse). L'acide 12-hydroxyoctadecanoïque (ou acide 12-hydroxystéarique, HSA) est un organogélifiant qui gélifie en formant un réseau 3D de fibres enchevêtrées. Les cires, telles que les cires de carnauba (Car) et de candelilla (Can) sont aussi des organogélifiants, mais qui gélifient en formant un réseau interconnecté de cristallites. Différentes huiles essentielles ont été gélifiées par trois organogélifiants. Les températures de gélification ont été déterminées. La volatilisation des huiles essentielles pures ou gélifiées a été mesurée par analyse thermogravimétrique (ATG) à une température fixée. Les trois huiles essentielles testées sont l'eucalyptus (Eucalyptus globulus, Phytosun aroms®), la lavande (Lavandula officinalis, Phytosun aroms®) et le thym (Thymus vulgaris, Phytosun aroms®). Le cas où une huile essentielle est diluée/dissoute dans un liquide organique non volatil a également été testé : le trioctanoate de glycéryle ou l'huile de soja ont été choisis.

Les organogélifiants testés sont l'acide 12-hydroxystéarique pur et des cires végétales, en particulier la cire de carnauba et de Candelilla. Ces deux cires végétales sont utilisées dans les industries cosmétiques et agroalimentaires. Ces cires végétales sont des mélanges complexes de composés naturels. La cire de carnauba contient principalement des esters d'acides gras (80-85 %), des alcools gras (10-15 %) des acides (3-6 %) et des chaines hydrocarbonées (1-3 %) . Une particularité de la cire de carnauba est la présence d'un fort taux d'esters de diols (environ 20 %), d'acides gras hydroxylés (environ 6 %) et d'acide cinnamique (environ 10 %) qui est un antioxydant. La cire de carnauba a point de fusion de 78 à 85 °C, c'est l'un des plus hauts parmi les cires d'origines naturelles. La cire de Candelilla est composée d'hydrocarbures (environ 50 %, de longueur de chaines de 29 à 33 atomes de carbones), d'esters, d'acides gras libres d'alcools et de résines. Son point de fusion est de 68 à 70 °C. Le principal composé de la cire de Candelilla est un alcane linéaire à 31 atomes de carbone, l'hentriacontane (Hent) ; ce composé pur est également testé comme organogélifiant.

### Mesure de la volatilisation par analyse thermogravimétrique ATG.

Un échantillon de masse connue est placé dans un creuset sous atmosphère contrôlée et à température contrôlée. La variation de masse de l'échantillon est mesurée sur 5 heures. La masse du résidu est exprimée en pourcentage de la masse initiale. Les expériences sont réalisées à 30 ou 40°C. Cette mesure en masse est précise, nous considéreront ici que l'erreur expérimentale est de 3%.

### RESULTATS

Après 5 heures d'expériences le résidu d'huile essentielle d'Eucalyptus est de 7% en masse; 93% de l'huile s'est volatilisée. Les huiles essentielles de lavande et de thym sont moins volatiles à 30°C puisque les résidus après 5 heures sont de 53 et 54 % respectivement.

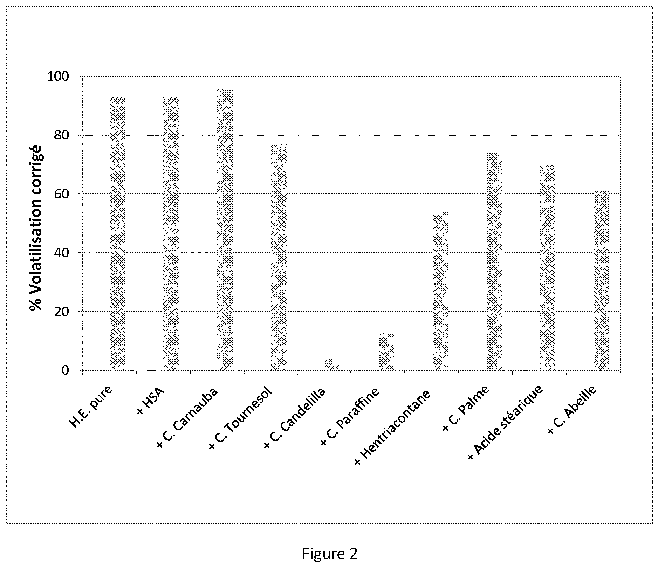

Lorsque l'huile essentielle est gélifiée nous devons procéder à une correction par rapport aux résultats bruts. Nous considérons que l'organogélifiant n'est pas volatil. Pour exprimer la proportion d'huile essentielle volatilisée par rapport à sa quantité initialement introduite il faut retirer la masse de l'organogélifiant. Dans la figure 2, l'huile essentielle d'eucalyptus est gélifiée avec 15% massique de HSA ; elle est volatilisée à 79% après 5 heures. Si l'on procède à la correction, le taux de volatilisation de l'huile essentielle est calculé à 93 %. Cette valeur peut être alors comparée à l'expérience de l'huile essentielle pure ; dans la suite de cet exposé ne seront reportées que les valeurs corrigées. On constate que l'organogel à base de HSA ne modifie pas les propriétés de volatilisation de l'huile essentielle d'eucalyptus : 93% de volatilisation, gélifiée ou non. Egalement, la cire de carnauba ne réduit pas la volatilisation de l'huile essentielle d'eucalyptus. Par contre la cire de candelilla a un effet très prononcé car il n'y a plus que 4% de l'huile qui s'est volatilisée. En résumé alors que la quasi-totalité de l'huile essentielle d'eucalyptus s'est volatilisée après 5 heures à 30°C, une fois gélifiée par la cire de candelilla elle ne se volatilise pratiquement plus.

Le pourcentage massique de cire de candelilla utilisé pour gélifier l'H.E. d'eucalyptus a une incidence sur la volatilisation du gel. En augmentant le pourcentage d'organogélifiant on retarde davantage la volatilisation de l'H. E.

Deux autres huiles essentielles (lavande et thym) ont été gélifiées par la cire de candelilla et testées en ATG.

L'huile essentielle de lavande se volatilise à 37% après 5 heures à 30°C. Une fois gélifiée avec la cire de candelilla, elle ne se volatilise plus qu'à 5% dans les mêmes conditions. Dans le cas de l'huile essentielle de thym, qui se volatilise à 43% après 5 heures à 30°C, la gélification par la cire de candelilla permet de réduire ces pertes à 8%.

Ces premières mesures de volatilisation ont clairement démontré que la gélification seule ne permet pas de réduire notoirement la volatilité d'une huile essentielle. Le résultat singulier obtenu lors de gélification par la cire de candelilla peut, dans une première approche, être expliqué par sa composition.

La cire de carnauba est extraite mécaniquement des feuilles d'un palmier à cire brésilien, et le HSA un produit de synthèse purifié. Par contre la cire de candelilla est obtenue de manière artisanale, à partir d'une euphorbe du Mexique (Euphorbia antisyphilitica) par une suite de procédés d'extraction chimiques. Il en résulte que cette cire contient, en plus de cires cuticulaires, des résidus de résine (environ 2% en masse) ; ce sont des acides résiniques comme par exemple l'acide abiétique.

Influence de résines naturelles sur la volatilité d'une huile essentielle et synergie avec l'organogélification :
L'effet particulièrement marqué de la gélification par la cire de candelilla a incité à tester l'effet de résines naturelles sur la réduction de la volatilité. Dans un premier temps, la résine de pin brute (pinus pinaster), la colophane (qui est le résidu solide obtenu après distillation de la térébenthine), et l'acide abiétique qui est un triterpénoide, principal composant de la colophane ont été utilisés. Le mode de réalisation avec l'acide abiétique est selon l'invention.

La gélification par l'hentriacontane (principal constituant de la cire de candelilla) ou le HSA seul ne permet pas de réduire la volatilisation de l'H. E (93%). On observe un effet de synergie de la résine très marqué lorsque l'H. E. est gélifiée avec l'hentriacontane (15% massique) puisque le taux de volatilisation est compris entre 27 et 12% selon la résine testée. Dans le cas de la gélification avec le HSA l'effet est moins marqué avec une volatilisation de 78% avec la colophane.

Dans le cas de la gélification de l'H.E. d'eucalyptus par le HSA (15% massique), la quantité de résine ajoutée a été testée. On constate que l'on peut réduire la volatilisation de l'H. E. en augmentant le taux de résine. Dans ce cas un taux de 2 à 8% permet de réduire la volatilisation de 78 à 56%.

L'ensemble de ces résultats souligne l'intérêt de l'invention dans le domaine du contrôle de la volatilité d'huiles essentielles ou de liquides organiques. La réduction de cette dernière grâce à l'ajout de résines naturelles, la gélification par la cire de candelilla ou l'ajout de résines à des organogels, permet d'étendre l'utilisation des huiles essentielles et diminuer l'inflammabilité de certains liquides organiques (eg. kérosène).

## Revendications

1. Composition sous la forme d'un organogel d'un liquide organique comprenant au moins un acide résinique, sous la forme acide ou sous la forme estérifiée, **caractérisée en ce que** la composition n'est pas une composition adhésive et **en ce que** l'acide résinique est choisi dans le groupe constitué par les acides résiniques abiétiques choisis dans le groupe constitué par l'acide abiétique, l'acide néoabiétique, l'acide déhydroabiétique, l'acide palustrique et leurs mélanges.

2. Composition selon la revendication 1 **caractérisée en ce que** l'organogel est composé d'un liquide organique, comprenant ou consistant en un liquide organique volatil, et d'un organogélifiant, le liquide organique volatil étant un liquide dont la pression de vapeur saturante à 20°C est supérieure à 0,01 mm de Hg, préférentiellement elle est comprise entre 0,01 et 20, de manière plus préférentielle entre 0,01 et 10, plus préférentiellement entre 0,01 et 5 (1 mm de Hg correspond à 133,32 Pa).

3. Composition selon la revendication 1 ou 2, **caractérisée en ce que** le liquide organique, comprenant ou consistant en un liquide organique volatil, est choisi dans le groupe constitué par les huiles essentielles, les solvants organiques, les phéromones à chaînes grasses, les hydrocarbures et leurs mélanges.

4. Composition selon la revendication 1 ou 2, **caractérisée en ce que** le liquide organique est un liquide organique volatil et est choisi dans le groupe constitué par les huiles essentielles, les liquides solvants organiques volatils, les phéromones à chaînes grasses, les hydrocarbures et leurs mélanges.

5. Composition selon l'une des revendications 1 à 3, **caractérisée en ce que** le liquide organique comprend un liquide organique volatil et un liquide organique non volatil choisi dans le groupe des huiles végétales, des huiles minérales, des huiles d'intérêt cosmétique et leurs mélanges.

6. Composition selon l'une quelconque des revendications 1 à 5, **caractérisée en ce que** l'organogélifiant est choisi dans le groupe constitué par les cires végétales, les cires animales, les cires de paraffine, les dérivés d'acide gras, les dérivés de sels d'acide gras et plus particulièrement l'acide 12-hydroxystéarique, utilisés seuls ou en mélange.

7. Composition selon la revendication 6, **caractérisée en ce que** la cire végétale est choisie dans le groupe constitué par la cire de Candelilla, cire de Carnauba, la cire de tournesol, la cire d'abeille, la cire de son de riz, la cire de canne à sucre, la cire de pépins de raisin, la cire de jojoba, la cire de graine de sorgho, la cire de ouiricuri, la cire de feuilles de pois, la cire de feuilles de pommes de terre, la cire d'Esparto, la cire d'Ocotillo, la cire de Balanophora, la cire d'attier, la cire de seigle de mer, la cire du laurier-cerise, la cire de chêne, la cire de mimosa, la cire de fleur d'oranger, la cire de jasmin, la cire de rose, la cire de chanvre, la cire d'abricot, la cire de tubéreuse, la cire d'orange, la cire d'amande douce la cire d'aiguilles de pin.

8. Composition selon l'une des revendications 1 à 7, **caractérisée en ce que** la quantité en poids d'organogélifiant dans l'organogel est comprise entre 1 et 30%.

9. Composition selon l'une quelconque des revendications précédentes, **caractérisée en ce que** l'acide résinique est sous la forme d'une résine végétale ou d'un mélange de résines végétales.

10. Composition selon la revendication 9, **caractérisée en ce que** la résine végétale est choisie dans le groupe constitué par la résine de pin, la résine de colophane, la résine de Sondarac, la résine Dammar, la gomme mastic et leurs mélanges.

11. Composition selon l'une quelconque des revendications précédentes, **caractérisée en ce que** la quantité en poids d'acide résinique dans l'organogel est comprise entre 0.5 et 10%.

12. Utilisation d'une composition selon l'une quelconque des revendications précédentes pour réduire la volatilité du liquide organique, comprenant ou consistant en un liquide organique volatil, contenu dans l'organogel.

13. Utilisation selon la revendication 12 dans laquelle le liquide organique, comprenant ou consistant en un liquide organique volatil, est un hydrocarbure inflammable afin de limiter les risques d'explosion.

14. Utilisation selon la revendication 12, dans laquelle le liquide organique, comprenant ou consistant en un liquide organique volatil, est une phéromone, en particulier une phéromone à chaîne grasse ou un mélange de phéromones, en particulier de phéromones à chaine grasse, pour la diffusion contrôlée de cette phéromone ou de ce mélange de phéromones.

15. Utilisation selon la revendication 12, dans laquelle le liquide organique, comprenant ou consistant en un liquide organique volatil, est choisi dans le groupe des liquides insecticides ou insectifuges, anti-fongiques, anti-viraux ou anti-bactériens pour la diffusion contrôlée de ce liquide organique.

16. Formulation insecticide ou insectifuge comprenant une composition selon l'une des revendications 1 à 11, dans laquelle le liquide organique, comprenant ou consistant en un liquide organique volatil, est doté de propriétés insecticides ou insectifuges.

17. Formulation anti-bactérienne comprenant une composition selon l'une des revendications 1 à 11, dans laquelle le liquide organique, comprenant ou consistant en un liquide organique volatil, est doté de propriétés antibactériennes.

18. Formulation anti-virale comprenant une composition selon l'une des revendications 1 à 11, dans laquelle le liquide organique, comprenant ou consistant en un liquide organique volatil, est doté de propriétés anti-virales.

19. Procédé de préparation d'une composition selon l'une des revendications 1 à 11 comprenant les étapes de :
a)Préparation d'un mélange comprenant un organogélifiant, un liquide organique, comprenant ou consistant en un liquide organique volatil, et liquide à température ambiante, et au moins un acide résinique choisi dans le groupe mentionné dans la revendication 1,
b)chauffage afin de solubiliser tous les ingrédients,
c)refroidissement à température ambiante du mélange afin de former un organogel.

## Patentansprüche

1. Zusammensetzung in Form eines Organogels aus einer organischen Flüssigkeit, die mindestens eine Harzsäure in Säureform oder in veresterter Form enthält, **dadurch gekennzeichnet, dass** die Zusammensetzung keine adhäsive Zusammensetzung ist und dass die Harzsäure ausgewählt ist aus der Gruppe bestehend aus abietischen Harzsäuren, die aus der Gruppe ausgewählt sind bestehend aus Abietinsäure, Neoabietinsäure, Dehydroabietinsäure, Palustrinsäure und Mischungen davon.

2. Zusammensetzung nach Anspruch 1, **dadurch gekennzeichnet, dass** das Organogel aus einer organischen Flüssigkeit, die eine flüchtige organische Flüssigkeit umfasst oder aus dieser besteht, und einem Organogeliermittel zusammengesetzt ist, wobei die flüchtige organische Flüssigkeit eine Flüssigkeit ist, deren Sättigungsdampfdruck bei 20°C größer als 0,01 mm Hg ist, vorzugsweise zwischen 0,01 und 20, noch bevorzugter zwischen 0,01 und 10, noch bevorzugter zwischen 0,01 und 5 liegt (1 mm Hg entspricht 133,32 Pa) .

3. Zusammensetzung nach Anspruch 1 oder 2, **dadurch gekennzeichnet, dass** die organische Flüssigkeit, die eine flüchtige organische Flüssigkeit umfasst oder daraus besteht, ausgewählt ist aus der Gruppe bestehend aus ätherischen Ölen, organischen Lösungsmitteln, Fettketten-Pheromonen, Kohlenwasserstoffen und Mischungen davon.

4. Zusammensetzung nach Anspruch 1 oder 2, **dadurch gekennzeichnet, dass** die organische Flüssigkeit eine flüchtige organische Flüssigkeit ist und ausgewählt ist aus der Gruppe bestehend aus ätherischen Ölen, flüchtigen flüssigen organischen Lösungsmitteln, Fettketten-Pheromonen, Kohlenwasserstoffen und Mischungen davon.

5. Zusammensetzung nach einem der Ansprüche 1 bis 3, **dadurch gekennzeichnet, dass** die organische Flüssigkeit eine flüchtige organische Flüssigkeit und eine nicht-flüchtige organische Flüssigkeit umfasst ausgewählt aus der Gruppe bestehend aus pflanzlichen Ölen, Mineralölen, Ölen von kosmetischem Interesse und Mischungen davon.

6. Zusammensetzung nach einem der Ansprüche 1 bis 5, **dadurch gekennzeichnet, dass** das Organogeliermittel ausgewählt ist aus der Gruppe bestehend aus pflanzlichen Wachsen, tierischen Wachsen, Paraffinwachsen, Fettsäurederivaten, Fettsäuresalzderivaten und insbesondere 12-Hydroxystearinsäure, allein oder in einer Mischung verwendet.

7. Zusammensetzung nach Anspruch 6, **dadurch gekennzeichnet, dass** das pflanzliche Wachs ausgewählt ist aus der Gruppe bestehend aus Candelillawachs, Carnaubawachs, Sonnenblumenwachs, Bienenwachs, Reiskleiewachs, Zuckerrohrwachs, Traubenkernwachs, Jojobawachs, Sorghumsamenwachs, Ouiricuriwachs, Erbsenblattwachs, Kartoffelblattwachs, Espartowachs, Ocotillowachs, Balanophorawachs, Zimtapfelwachs, Strandroggenwachs, Kirschlorbeerwachs, Eichenwachs, Mimosenwachs, Orangenblütenwachs, Jasminwachs, Rosenwachs, Hanfwachs, Aprikosenwachs, Tuberosenwachs, Orangenwachs, Süßmandelwachs und Kiefernnadelwachs.

8. Zusammensetzung nach einem der Ansprüche 1 bis 7, **dadurch gekennzeichnet, dass** der Gewichtsanteil des Organogeliermittels zwischen 1 und 30% liegt.

9. Zusammensetzung nach einem der vorstehenden Ansprüche, **dadurch gekennzeichnet, dass** die Harzsäure in Form eines pflanzlichen Harzes oder eines Gemisches von pflanzlichen Harzen vorliegt.

10. Zusammensetzung nach Anspruch 9, wobei das pflanzliche Harz ausgewählt ist aus der Gruppe bestehend aus Kiefernharz, Kolophoniumharz, Sondaracharz, Dammarharz, Gummimastix und Mischungen davon.

11. Zusammensetzung nach einem der vorstehenden Ansprüche, **dadurch gekennzeichnet, dass** der Gewichtsanteil der Harzsäure im Organogel zwischen 0,5 und 10% liegt.

12. Verwendung einer Zusammensetzung nach einem der vorstehenden Ansprüche zur Verringerung der Flüchtigkeit der in dem Organogel enthaltenen organischen Flüssigkeit, die eine flüchtige organische Flüssigkeit umfasst oder daraus besteht.

13. Verwendung nach Anspruch 12, wobei die organische Flüssigkeit, die eine flüchtige organische Flüssigkeit umfasst oder daraus besteht, ein entzündlicher Kohlenwasserstoff ist, um die Explosionsgefahr zu begrenzen.

14. Verwendung nach Anspruch 12, **dadurch gekennzeichnet, dass** die organische Flüssigkeit, die eine flüchtige organische Flüssigkeit umfasst oder daraus besteht, ein Pheromon, insbesondere ein Fettketten-Pheromon oder ein Gemisch von Pheromonen, insbesondere Fettketten-Pheromonen, zur kontrollierten Freisetzung dieses Pheromons oder Pheromongemisches ist.

15. Verwendung nach Anspruch 12, wobei die organische Flüssigkeit, die eine flüchtige organische Flüssigkeit umfasst oder daraus besteht, ausgewählt ist aus der Gruppe der insektentötenden oder insektenvertreibenden, antifungalen, antiviralen oder antibakteriellen Flüssigkeiten zur kontrollierten Freisetzung dieser organischen Flüssigkeit.

16. Insektentötende oder insektenvertreibende Rezeptur umfassend eine Zusammensetzung nach einem der Ansprüche 1 bis 11, wobei die organische Flüssigkeit, die eine flüchtige organische Flüssigkeit umfasst oder daraus besteht, mit insektentötenden oder insektenvertreibenden Eigenschaften ausgestattet ist.

17. Antibakterielle Rezeptur umfassend eine Zusammensetzung nach einem der Ansprüche 1 bis 11, wobei die organische Flüssigkeit, die eine flüchtige organische Flüssigkeit umfasst oder daraus besteht, mit antibakteriellen Eigenschaften ausgestattet ist.

18. Antivirale Rezeptur umfassend eine Zusammensetzung nach einem der Ansprüche 1 bis 11, wobei die organische Flüssigkeit, die eine flüchtige organische Flüssigkeit umfasst oder daraus besteht, mit antiviralen Eigenschaften ausgestattet ist.

19. Verfahren zur Herstellung einer Zusammensetzung nach einem der Ansprüche 1 bis 11, umfassend die Schritte:
a) Herstellen eines Gemisches, das ein Organogeliermittel, eine organische Flüssigkeit, die eine flüchtige organische Flüssigkeit umfasst oder daraus besteht und flüssig ist bei Raumtemperatur, und mindestens eine Harzsäure ausgewählt aus der in Anspruch 1 genannten Gruppe umfasst,
b) Erhitzen, um alle Bestandteile aufzulösen,
c) Abkühlen der Mischung auf Raumtemperatur zur Bildung eines Organogels.

## Claims

1. Composition in the form of an organogel of an organic liquid comprising at least one resin acid, in the acid form or in the esterified form, **characterized in that** the composition is not an adhesive composition and **in that** the resin acid is selected from the group consisting of abietic resin acids selected from the group consisting of abietic acid, neoabietic acid, dehydroabietic acid, palustric acid and mixtures thereof.

2. Composition according to claim 1, **characterized in that** the organogel is composed of an organic liquid, comprising or consisting of a volatile organic liquid, and of an organogelator, the volatile organic liquid being a liquid whose saturated vapor pressure at 20°C is greater than 0.01 mmHg, preferentially it is comprised between 0.01 and 20, more preferentially between 0.01 and 10, more preferentially between 0.01 and 5 (1 mmHg corresponds to 133.32 Pa).

3. Composition according to claim 1 or 2, **characterized in that** the organic liquid, comprising or consisting of a volatile organic liquid, is selected from the group consisting of essential oils, organic solvents, fatty chain pheromones, hydrocarbons and mixtures thereof.

4. Composition according to claim 1 or 2, **characterized in that** the organic liquid is a volatile organic liquid and is selected from the group consisting of essential oils, volatile organic solvent liquids, fatty chain pheromones, hydrocarbons and mixtures thereof.

5. Composition according to any one of claims 1 to 3, **characterized in that** the organic liquid comprises a volatile organic liquid and a non-volatile organic liquid selected from the group consisting of vegetable oils, mineral oils, oils of cosmetic interest and mixtures thereof.

6. Composition according to any one of claims 1 to 5, **characterized in that** the organogelator is selected from the group consisting of vegetable waxes, animal waxes, kerosene waxes, fatty acid derivatives, fatty acid salt derivatives and more particularly 12-hydroxystearic acid, used alone or in mixture.

7. Composition according to claim 6, **characterized in that** the vegetable wax is selected from the group consisting of candelilla wax, carnauba wax, sunflower seed wax, beeswax, rice bran wax, sugar cane wax, grape seed wax, jojoba wax, sorghum seed wax, ouricury wax, pea leaf wax, potato leaf wax, esparto wax, ocotillo wax, Balanophora wax, Annona squamosa wax, sea rye wax, cherry laurel wax, oak wax, mimosa wax, orange blossom wax, jasmine wax, rose wax, hemp wax, apricot wax, tuberose wax, orange wax, sweet almond wax or pine needle wax.

8. Composition according to any one of claims 1 to 7, **characterized in that** the quantity by weight of organogel is comprised between 1 and 30%.

9. Composition according to any one of the preceding claims, **characterized in that** the resin acid is in the form of a plant resin or a mixture of plant resins.

10. Composition according to claim 9, **characterized in that** the plant resin is selected from the group consisting of pine resin, rosin, Sondarac resin, Dammar resin, mastic gum and mixtures thereof.

11. Composition according to any one of the preceding claims, **characterized in that** the quantity by weight of resin acid in the organogel is comprised between 0.5 and 10%.

12. Use of a composition according to any one of the preceding claims to reduce the volatility of the organic liquid, comprising or consisting of a volatile organic liquid, contained in the organogel.

13. Use according to claim 12, in which the organic liquid, comprising or consisting of a volatile organic liquid, is an inflammable hydrocarbon in order to limit the risks of explosion.

14. Use according to claim 12, in which the organic liquid, comprising or consisting of a volatile organic liquid, is a pheromone, in particular a fatty chain pheromone or a mixture of pheromones, in particular fatty chain pheromones, for the controlled release of this pheromone or this mixture of pheromones.

15. Use according to claim 12, in which the organic liquid, comprising or consisting of a volatile organic liquid, is selected from the group of liquid insecticides or insect repellents, antifungal, antiviral or antibacterial liquids for the controlled diffusion of this organic liquid.

16. Insecticide or insect repellent formulation comprising a composition according to one of claims 1 to 11, in which the organic liquid, comprising or consisting of a volatile organic liquid, is endowed with insecticidal or insect repellent properties.

17. Antibacterial formulation comprising a composition according to one of claims 1 to 11, in which the organic liquid, comprising or consisting of a volatile organic liquid, is endowed with antibacterial properties.

18. Antiviral formulation comprising a composition according to one of claims 1 to 11, in which the organic liquid, comprising or consisting of a volatile organic liquid, is endowed with antiviral properties.

19. Preparation method for a composition according to one of claims 1 to 11 comprising the steps of:
a) Preparation of a mixture comprising an organogelator, an organic liquid, comprising or consisting of a volatile organic liquid, and liquid at room temperature, and at least one resin acid selected from the group mentioned in claim 1,
b) heating in order to solubilize all the ingredients,
c) cooling of the mixture to room temperature in order to form an organogel.
